# EUROPEAN PATENT SPECIFICATION

(11) **EP 0 282 179 B1**
(45) Date of publication and mention of the grant of the patent: **10.05.1995**
(21) Application number: 88301296.5
(22) Date of filing: 17.02.1988
(51) Int. Cl.: A61K 39/175

(54) **Canine distemper virus vaccine**
Staupevirus-Impfstoff
Vaccin contre le virus de la maladie des chiens

(30) Priority: 09.03.1987 US 23814
(43) Date of publication of application: 14.09.1988
(60) Divisional application: 92109639.2
(73) Proprietor: Bayer Corporation, Pittsburgh, PA 15219-2502 (US)
(72) Inventor: Bass, Edmund P., Menlo Park, CA 94024 (US); Kelsey, William H., West Des Moines, IA 50265 (US); McFarland, Michael D., Des Moines, IA 50317 (US)
(74) Representative: Thomson, Paul Anthony

(56) References cited:
- US-A- 4 545 987
- US-A- 4 693 981
- CHEMICAL ABSTRACTS, vol. 87, no. 11, 12 September 1977, Columbus, OH (US); J.E. HEARST et al., p. 98, no. 78962f/
- CHEMICAL ABSTRACTS, vol. 100, no. 3, 16 January 1984, Columbus, OH (US); C.V. HANSON, p. 269, no. 20043c/
- AN INTRODUCTION TO VETERINARY IMMUNOLOGY, I. Tizard, 2nd ed., W.B. Saunders Co., Philadelphia, PA (US), 1982; pp. 182-186/
- FIELDS VIROLOGY, 2nd ed., B.N. Fields & D.M. Knipe (eds.), Raven Press, New York, NY (US), 1990; chapter 19, pp. 480-486/
- F. FENNER et al., "Veterinary Virology", vol. 14, Academic Press Inc., New York, NY (US); pp. 274-276/
- ADVANCES IN VETERINARY & COMPARATIVE MEDICINE, vol. 33, 1989; p. 30/
- ARCH. VIROLOGY, vol. 47, 1975; pp. 47-56/
- CORNELL VET., vol. 55, 1963; pp. 3-8/
- JAWMA, vol. 179, no. 11, 1981; pp. 1170-1174/
- JAWMA, vol. 180, no. 2, 1982; pp. 137-139/
- JAWMA, vol. 181, no. 10, 1982; pp. 1142-1149/
- JAWMA, vol. 186, no. 11, 1985; pp. 1217-1219/
- JOURNAL OF CLINICAL MICROBIOLOGY, vol. 11, 1980; pp. 120-122/
- JOURNAL OF CLINICAL MICROBIOLOGY, vol. 8, 1978; pp. 604-611/
- JOURNAL OF GEN. VIROLOGY, vol. 41, 1978; pp. 385-393/
- JOURNAL OF GEN. VIROLOGY, vol. 67, 1986; pp. 1381-1392/
- JOURNAL OF VIROLOGY, vol. 58, 1986; pp. 536-541/
- JOURNAL ZOO AN. MED., vol. 14, 1983; pp. 66-71/
- THE VETERINARY RECORD, 21 February 1981; p. 171/
- VETERINARY PATHOLOGY, vol. 11, 1974; pp. 301-312/

## Description

The present invention is directed to a new vaccine composition for canine distemper virus. It is also directed to a method for inactivating canine distemper virus, such that the virus while inactivated, nevertheless substantially retains its immunogenicity.

Canine distemper is one of the most serious viral diseases of dogs. The disease is highly contagious and is characterized by severe morbidity and high mortality. Modified live canine distemper virus vaccines are currently available in the United States. They do provide protective immunity to distemper, but such vaccines may revert to virulence, may cause immunosupression following vaccination, and have been shown to cause mortality in non-canine species. There is therefore a real need for an efficacious, inactivated canine distemper virus vaccine, which eliminates the serious problems associated with modified live canine distemper virus vaccines.

J. Clin. Microbiol., 11(1980) pages 120-122 and Arch. Virol., 47(1975) pages 47-56 respectively describe the use of binary ethyleneimine as an inactivant for foot-and-mouth disease virus and rabies virus with a view to vaccine production.

An object of the present invention is to develop an inactivated canine distemper virus vaccine which substantially eliminates the serious problems, and risks, associated with modified live canine distemper virus vaccines.

According to the present invention there is provided a vaccine composition for animals susceptible to infection by canine distemper virus (CDV), said composition comprising an immunologically effective amount of an inactivated canine distemper virus obtainable by an inactivation procedure which allows the virus to retain its immunogenicity and a non-toxic pharmaceutically acceptable immunologic adjuvant, characterized in that the inactivation procedure comprises exposure of the virus to an inactivating effective amount of binary ethyleneimine.

Also according to the present invention there is provided a process of inactivating canine distemper virus which allows the virus to substantially retain its immunogenicity, comprising exposing the virus to an inactivating effective amount of binary ethyleneimine at a temperature ranging from -10°C to 40°C for a time sufficient to render the virus non-infectious.

In this invention, inactivated vaccines are manufactured for the protection of mammalian species, in particular the canine, from severe clinical signs and mortality resulting from infection with canine distemper virus (CDV). The vaccines are inactivated by the addition of binary ethyleneimine to virus fluids. The resulting inactivated virus preparation may be stored until used for vaccination.

Any strain of CDV may be inactivated for use in this invention. Of particular interest is the Rockborn strain, but other strains such as Snyder-Hill, Onderstepoort, etc. may be used.

In preparing the vaccine, virulent CDV is grown in cultured mammalian cells in conventional virus growth conditions. Examples of cells used are the DKF (dog kidney) cell line, and tertiary canine kidney cells. The host cells may be seeded with virus at the time of cell planting, or with a CDV-containing media change when the cell monolayer is 90-100% confluent. The multiplicity of infection (MOI) ratio may be from about 0.001 to about 0.05, preferably about 0.01. Any appropriate mammalian cell growth medium, such as Eagle's Minimum Essential Medium, containing bovine serum at from 0-10%, and antibiotics, is used to produce the viral fluids.

Infected cell cultures are maintained in a temperature range of from about 35°C to about 40°C for from 2 to about 7 days post seeding at which time virus-containing fluids are harvested. The CDV infected cultures may be refed with cell growth medium, which may be harvested after an additional 2 to 5 days incubation. Virus fluids are harvested into sterile containers and may be clarified by filtration. The virus fluids may further be concentrated, either prior to or post inactivation, using conventional ultrafiltration technology (e.g. Millipore Pellicon system XX42ASY60 with a cassette having a normal exclusion limit of 10⁵ daltons such as Millipore PTHK000C5). 'Millipore' is a Registered Trade Mark.

The virulent canine distemper virus after harvest is ready for inactivation. The inactivation must be by a technique which destroys the virus' ability to replicate, but at the same time does not destroy the immunological activity of the inactivated virus. Heretofore there never has been a successful inactivated canine distemper virus vaccine. In this invention there is provided a virus which is both inactivated and at the same time retains immunogenicity.

As used herein, the term "inactivated" refers to previously virulent virus which have undergone treatment to inactivate, kill, or otherwise modify them to substantially eliminate their virulent properties while yet retaining their characteristic property of immunogenicity.

In accordance with this invention, the technique for inactivating and retaining immunogencity comprises inactivating CDV fluids with binary ethyleneimine (BEI). BEI is prepared by adding 1.0 N sodium hydroxide to a 5% solution of bromoethylamine hydrobromide followed by incubation at, for example, about 37°C for 60 minutes. The resulting BEI inactivant is added to virus fluids to a final concentration of from about 0.005 to about 0.01 molar. The inactivating virus fluids are incubated at from about 4°C to about 38°C for an interval of from about 24 to about 72 hours. The BEI is subsequently neutralized, for example, by the addition of a 50% solution of sodium thiosulfate in sufficient quantity to equal ten times the molarity of the BEI. It should be understood that the above specific technique for inactivation for binary ethyleneimine is exemplary only. That is, there may be some modifications in concentrations, temperature ranges, etc. such that the inactivation is still effectively accomplished. Generally speaking, the temperature range during the binary ethyleneimine inactivation should be from about -10°C to about 40°C.

In preparation of the vaccine, the inactivated collected canine distemper virus is mixed with a nontoxic pharmaceutically acceptable immunologic adjuvant such as Freund's complete adjuvant, aluminum hydroxide or oil-in-water or water-in-oil adjuvants. Also, Interleukin-1, or other immuno-enhancing substances may be used. Preparation of the vaccine entails mixing the inactivated virus fluids with an adjuvant. The amount of virus per dose of vaccine is equivalent to from approximately 10⁴ to 10⁸ 50% cell culture infective doses per ml (CCID₅₀/ml). In addition, other viruses or bacteria may be included such as, but not limited to, canine adenovirus type 2, canine parainfluenzae virus, canine parvovirus, Leptospira canicola and Leptospira icterohaemorrhagiae. The vaccine may be administered subcutaneously or intramuscularly. The vaccine inoculation volume will range from about 0.5 ml to 4 ml. Normally 2 injections are given at from 1 to 3 week intervals.

Preferably the dosage of the inactivated canine distemper virus is from about 10⁴ to about 10⁸ CCID₅₀/ml, that is, cell culture infective dose/ml. More preferably, within the range of from about 10⁵ to about 10⁷ CCID₅₀/ml and most preferably within the range of about 10^{5.8} to about 10^{6.2} CCID₅₀/ml.

By way of information there is now illustrated how the canine distemper virus was grown and assayed.

### A. Production of Virus and Tissue Culture

Canine cells (DKF), a dog kidney cell line, or (DK) tertiary dog kidney cells are grown as monolayers in plastic cell culture vessels in Eagle's Minimum Essential Medium with Earl's salts and non-essential amino acids (MEN) supplemented with 5% heat inactivated fetal bovine serum or 5% heat inactivated calf serum. Cell cultures are used to produce live CDV from master seed virus. Cells are grown in culture vessels to 80% to 100% confluency (approximately 2 x 10⁵ cells per cm² of growth surface area) using standard mammalin cell culture techniques. Generally, plastic roller bottles (e.g. Corning® No. 25140-850) with a growth surface area of 850 cm² containing 100 ml of MEN supplemented with 5% fetal bovine serum and 1 x 10⁸ to 2 x 10⁸ cells/bottle are used for virus production although other permissive cells, other culture vessels, other culture media or other supplements may be used. The cell cultures are initiated by seeding approximately 1 x 10⁷ to 2 x 10⁷ cells into 100 ml of growth medium in a roller bottle on a roller bottle rotator at 0.25 to 2 rpm at 35° to 38°C. The cultures are grown to 80% to 100% confluency over a seven to ten day period with a medium change every three to five days.

When the monolayers are 80% to 100% confluent the culture medium is removed. Two hundred ml of MEN supplemented with 0.5% lactalbumin hydrolysate (e.g. GIBCO® 670-1800) is added per roller bottle. Monolayers are infected with 10⁵ to 10⁶ CCID₅₀ of CDV per roller bottle. The multiplicity of infection (MOI) is approximately 0.01. The MOI may range from 0.001 to 0.05. The post-infection incubation is at 35° to 38°C with rotation. Two to five days post-infection, CDV cytopathic effect (CPE) is evident. Two days post-infection, the CPE is characterized by the appearance of some vacuolated cells and some multi-nucleated giant cells. On day five post-infetion, giant cells are more numerous. On day five post-infection, the medium containing virus is harvested from the roller bottle/ The roller botttle is fed 200 ml fresh MEN supplemented with 0.5% LAH and incubated at 35° to 38°C with rotation for an additional three days. During the six to eight day post-infection period, the CPE progresses to the point that many cells are shed into the medium. By day eight post-infection, 50% to 90% CPE is evident. On day eight post-infection, the medium containing virus is harvested.

The virus preparation may be concentrated by ultra-filtration using a Pellicon cassette system (Millipore XX42ASY60) with a cassette having a nominal exclusion of 10⁵ daltons (Millipore PTHK000C5). The Pellicon, cassette system is sterilized by filling the assembled unit with 1 N NaOH and incubating the unit 12 to 14 hours at room temperature. The NaOH solution is pumped out of the cassette system and the system is flushed with two to four liters of sterile H₂O. The assembly and operation of the Pellicon system are in accordance with the instruction furnished by the manufacturer. All steps in the concentration are performed aseptically.

### B. Virus Assay

Ten-fold serial dilutions of a virus sample are made by adding 0.1 ml of the virus sample to 0.9 ml of MEN supplemented with 5% Fⁱ. Eight chamber Lab-Tek slides are seeded with 0.4 ml/chamber of DKF cells at 1.5 x 10⁵ cells/ml in MEN supplemented with 5% Fⁱ (approximately 6 x 10⁴ cells/chamber). Immediately following seeding of cells, 0.1 ml aliquots of each serial dilution are added to each of five chambers. The slides are incubated at 35° to 38°C in 5% CO₂ in air for six days. On day six, remove the medium, plastic chamber and gasket from the slide. Rinse slides once in PBS, fix in acetone at -20°C for 20 minutes and air dry. Anti-CDV FITC conjugate (0.1 ml) is placed on each slide and a coverslip is placed on the monolayer to spread the conjugate over the entire cell sheet. Slides are incubated in a high humidity incubator for 30 minutes at 35° to 37°C. Coverslips are removed in 3x3 minute rinses in PBS at room temperature. Slides are counter stained for 90 seconds in Evans blue, rinsed twice for 3 minutes in PBS, rinsed in distilled water and placed in a modified X-ray dryer until dry. Coverslips are mounted on slides using mounting fluid (80% glycerol in PBS, v/v). Cells are examined for fluorescence tyical of CDV which constitutes a positive response. End point is calculated by the method of Reed and Muench.

The following examples are offered to further illustrate but not limit both the composition of the invention, the method of preparation of the composition of the invention, and the method of use of the composition of the invention to immunize mammals against canine distemper virus.

### Example 1

### BEI Inactivation

A 0.2 molar solution of bromoethylamine hydrobomide (BEA) is prepared by dissolving 3.55 grams in 71 ml distilled water. 56.8 ml of BEA solution is added to 14.2 ml 1.0 normal sodium hydroxide solution. Binary ethyleneimine (BEI) is formed by incubating the above mixture for 1 hour at 36°C. 1343 ml of harvested canine distemper virus fluids are brought to 37°C, to which the BEI solution is added while the suspension is stirred. The inactivation is continued by incubation at 37° for 28 hours. The BEI is then neutralized by the addition of 46.2 ml of a 50% solution of sodium thiosulfate.

To confirm complete viral inactivation two ml of the inactivated virus fluids are tested for the presence of residual live virus by inoculation onto a 150 cm² monolayer of dog kidney cells. Inoculated cells are incubated for 7 days, then are subpassaged. If cytopathic effects due to CDV infection are not observed during the first or second in vitro passages, it is concluded virus fluids do not contain residual live CDV.

### Example 2

### Testing of Immunological Response

Binary ethyleneimine inactivated CD virus of the preferred Rockborn strain was used to formulate vaccine with adjuvant B. The binary ethyleneimine inactivation procedure was performed as described in Example 1.

Five dogs, (CDV sero-negative) identified as numbers 29, 65, 68, 71 and 72, were vaccinated twice at a 21-day interval with a 1.0 ml dose of BEI-inactivated CDV vaccine containing 10^{5.9} CCID₅₀ of the Rockborn strain of CDV and 50% Adjuvant B. Serum was collected for serologic evaluation at the time of inoculations and at 14 days following the second vaccination.

In vitro virus neutralization of CDV by sera collected was examined using a 50% plaque reduction test (Table 1). Positive and negative control sera behaved as expected.

**Table 1**

| VIRUS NEUTRALIZATION DATA FROM DOGS VACCINATED WITH BEI-INACTIVATED CDV | | | |
|---|---|---|---|
| Animal # | Antibody Response Day of Test | | |
| | 0 | 21 | 35 |
| 29 | <4 | 18 | 162 |
| 65 | <4 | <4 | 54 |
| 68 | <4 | 18 | 54 |
| 71 | <4 | 2 | 54 |
| 72 | <4 | 18 | 54 |

Table 1 shows anti-canine distemper serum neutralizing antibody as determined by a constant virus/serum dilution in vitro neutralization test. This is a conventional procedure and for details concerning it, see Ross and Friedman's Manual of Clinical Immunology. Table 1 provides the antibody titers from the five vaccinated dogs and demonstrates successful immunization.

The above methods for viral inactivation, and testing of immunological response demonstrates both preparation and successful use of the canine distemper vaccine of this invention. It also represents the first ever efficacious successful inactivated canine distemper vaccine. It therefore can be seen that the invention accomplishes at least all of its stated objectives.

In a co-pending application, EP-A-0 506 147, which has been divided out of the present invention, there is disclosed a vaccine composition for animals susceptible to infection by canine distemper virus (CDV), said composition comprising an immunologically effective amount of an inactivated canine distemper virus which, although inactivated, retains its immunogenicity and a non-toxic pharmaceutically acceptable immunologic adjuvant, characterized in that the canine distemper virus is inactivated by an inactivation procedure comprising exposing the virus to an inactivating effective amount of long wave length ultraviolet light, ultraviolet radiation or gamma radiation in the presence of a furocoumarin. There is also disclosed a method for preparing such virus and the use of such compositions for protecting mammals from infection caused by canine distemper virus.

## Claims

1. A vaccine composition for animals susceptible to infection by canine distemper virus (CDV), said composition comprising an immunologically effective amount of an inactivated canine distemper virus obtainable by an inactivation procedure which allows the virus to retain its immunogenicity and a non-toxic pharmaceutically acceptable immunologic adjuvant, characterized in that the inactivation procedure comprises exposure of the virus to an inactivating effective amount of binary ethyleneimine.

2. A vaccine composition according to claim 1, characterized in that the amount of the inactivated canine distemper virus is from 10⁴ to 10⁸ CCID₅₀/ml.

3. A vaccine composition according to claim 2, characterized in that the amount of the virus is from 10⁵ to 10⁷ CCID₅₀/ml.

4. A vaccine composition according to claim 3, characterized in that the amount of the virus is from 10^{5.8} to 10^{6.2} CCID₅₀/ml.

5. A vaccine composition according to any preceding claim, characterized in that the adjuvant comprises Freund's complete adjuvant, aluminum hydroxide or oil-in-water or water-in-oil adjuvants.

6. A vaccine composition according to any preceding claim, characterized in that the canine distemper virus is the Rockborn, Snyder-Hill or Onderstepoort strain.

7. A process of inactivating canine distemper virus which allows the virus to substantially retain its immunogenicity, comprising exposing the virus to an inactivating effective amount of binary ethyleneimine at a temperature ranging from -10°C to 40°C for a time sufficient to render the virus non-infectious.

8. A process according to claim 7, characterized in that the canine distemper virus is grown in cultured mammalian host cells prior to inactivation.

9. The use of a vaccine composition according to any of claims 1 to 6 for the manufacture of a medicament for protecting mammals from infection caused by canine distemper virus.

## Patentansprüche

1. Impfstoffzusammensetzung für Tiere, die anfällig sind gegen eine Infektion mit dem Hundestaupevirus (CDV), wobei diese Zusammensetzung eine immunologisch wirksame Menge eines inaktivierten Hundestaupevirus enthält, erhältlich durch ein Inaktivierungsverfahren, das es erlaubt, daß der Virus seine Immunität erzeugende Eigenschaft behält, sowie einen nicht toxischen pharmazeutisch verträglichen immunologischen Hilfsstoff enthält,
dadurch gekennzeichnet, daß das Inaktivierungsverfahren die Einwirkung einer für die Inaktivierung wirksamen Menge eines binären Ethylenimins auf den Virus umfaßt.

2. Impfstoffzusammensetzung nach Anspruch 1, dadurch gekennzeichnet, daß die Menge an inaktiviertem Hundestaupevirus zwischen 10⁴ und 10⁸ CCID₅₀/ml beträgt.

3. Impfstoffzusammensetzung nach Anspruch 2, dadurch gekennzeichnet, daß die Menge des Virus von 10⁵ bis 10⁷ CCID₅₀/ml beträgt.

4. Impfstoffzusammensetzung nach Anspruch 3, dadurch gekennzeichnet, daß die Menge des Virus zwischen 10^{5,8} und 10^{6,2} CCID₅₀/ml beträgt.

5. Impfstoffzusammensetzung gemäß einem der vorhergehenden Ansprüche, dadurch gekennzeichnet, daß der Hilfsstoff einen vollständigen Hilfsstoff nach Freund umfaßt, Aluminiumhydroxid oder Öl-in-Wasser oder Wasser-in-Öl Hilfsstoffe.

6. Impfstoffzusammensetzung gemäß einem der vorhergehenden Ansprüche, dadurch gekennzeichnet, daß der Hundestaupevirus ein solcher vom Rockborn-, Snyder-Hill- oder Onderstepoorttypus ist.

7. Verfahren zur Inaktivierung von Hundestaupevirus, das es erlaubt, daß der Virus im wesentlichen seine Immunität erzeugende Eigenschaft behält, umfassend die Einwirkung einer für die Inaktivierung wirksamen Menge eines binären Ethylenimins auf den Virus bei einer Temperatur im Bereich von - 10°C bis 40°C für eine Zeitdauer, die ausreicht, um den Virus nicht infektiös werden zu lassen.

8. Verfahren nach Anspruch 7, dadurch gekennzeichnet, daß der Hundestaupevirus in kultivierten Säugetierwirtszellen aufgezogen wird, bevor die Inaktivierung erfolgt.

9. Verwendung einer Impfstoffzusammensetzung gemäß einem der Ansprüche 1 bis 6 für die Herstellung eines Medikaments für den Schutz von Säugetieren gegen Infektion mit Hundestaupevirus.

## Revendications

1. Une composition de vaccin pour des animaux sensibles à l'infection par le virus de la maladie de Carré, cette composition comprenant une quantité immunologiquement efficace d'un virus de la maladie de Carré inactivé obtenu par une méthode d'inactivation permettant au virus de conserver son immunogénicité et un adjuvant immunologique non toxique pharmaceutiquement acceptable, caractérisée en ce que la méthode d'inactivation consiste à exposer le virus à une quantité inactivante efficace d'éthylèneimine binaire.

2. Une composition de vaccin selon la revendication 1, caractérisée en ce que la quantité du virus de la maladie de Carré inactivé est comprise entre 10⁴ et 10⁸ DICC₅₀/ml.

3. Une composition de vaccin selon la revendication 2, caractérisée en ce que la quantité du virus est comprise entre 10⁵ et 10⁷ DICC₅₀/ml.

4. Une composition de vaccin selon la revendication 3, caractérisée en ce que la quantité du virus est comprise entre 10^{5,8} et 10^{6,2} DICC₅₀/ml.

5. Une composition de vaccin selon l'une ou l'autre des revendications qui précèdent, caractérisée en ce que l'adjuvant comprend un adjuvant complet de Freund, de l'hydroxyde d'aluminium ou des adjuvants à base d'huile dans l'eau ou d'eau dans l'huile.

6. Une composition de vaccin selon l'une ou l'autre des revendications qui précèdent, caractérisée en ce que le virus de la maladie de Carré est la souche de Rockborn, de Snyder-Hill ou d'Onderstepoort.

7. Un procédé d'inactivation du virus de la maladie de Carré, qui permet au virus de conserver la quasi-totalité de son immunogénicité, consistant à exposer le virus à une quantité inactivante efficace d'éthylèneimine binaire à une température comprise entre -10°C et 40°C pendant un temps suffisant pour rendre le virus non infectieux.

8. Un procédé selon la revendication 7, caractérisé en ce que le virus de la maladie de Carré se développe dans des cellules-hôtes de mammifère cultivées avant l'inactivation.

9. L'utilisation d'une composition de vaccin selon l'une ou l'autre des revendications 1 à 6 pour la fabrication d'un médicament destiné à protéger des mammifères de l'infection provoquée par le virus de la maladie de Carré.
